# EUROPEAN PATENT SPECIFICATION

(11) **EP 2 295 049 B1**
(45) Date of publication and mention of the grant of the patent: **26.11.2014**
(21) Application number: 10174280.7
(22) Date of filing: 07.09.2006
(51) Int. Cl.: A61K 31/137, A61K 31/661, A61K 45/06, A61P 25/02, A61P 25/00, A61P 27/02

(54) **Treatment of autoimmune diseases**
Behandlung von Autoimmunkrankheiten
Traitement de maladies auto-immunes

(30) Priority: 09.09.2005 US 715990 P
(43) Date of publication of application: 16.03.2011
(62) Divisional of application: 06793341.6
(73) Proprietor: Novartis AG, 4056 Basel (CH)
(72) Inventor: Albert, Rainer, 4051, Basel (CH); Cooke, Nigel Graham, 4104, Oberwil (CH); Nuesslein-Hildesheim, Barbara, 79591, Eimeldigen (DE); Weiler, Sven, 79539, Loerrach (DE)
(74) Representative: von Sprecher, Georg

(56) References cited:
- EP-A- 1 602 660
- EP-A1- 1 431 284
- EP-A1- 1 772 145
- WO-A2-2004/028521
- WO-A2-2005/014525
- SHIMIZU, HISASHI ET AL: "KRP-203, a Novel Synthetic Immunosuppressant, Prolongs Graft Survival and Attenuates Chronic Rejection in Rat Skin and Heart Allografts", CIRCULATION , 111(2), 222-229 CODEN: CIRCAZ; ISSN: 0009-7322, 18 January 2005 (2005-01-18), XP002408357,
- BILLICH A ET AL: "Phosphorylation of the Immunomodulatory Drug FTY720 by Sphingosine Kinases", JOURNAL OF BIOLOGICAL CHEMISTRY, AMERICAN SOCIETY FOR BIOCHEMISTRY AND MOLECULAR BIOLOGY, US, vol. 278, no. 48, 28 November 2003 (2003-11-28), pages 47408-47415, XP003019924, ISSN: 0021-9258, DOI: 10.1074/JBC.M307687200
- 'KRP203' THOMSON REUTERS INTEGRITY

## Description

### Field of the Invention

The present invention relates to an amino alcohol and phosphate derivatives thereof, for use to treat particular autoimmune diseases, such as amyotrophic lateral sclerosis and uveitis.

### Background of the Invention

Multiple sclerosis is a chronic inflammatory disease of the central nervous system (CNS) with an unknown pathophysiological cause. Clinical manifestations are associated with the infiltration of the central nervous system by immune-competent cells. Specific T cell populations directed towards neuroantigens, such as myelin basic protein, can be demonstrated in the periphery. This suggests the involvement of an autoimmune response in the development of the disease Although there is no specific treatment for this T cell-mediated autoimmune disorder, patients receive immunosuppressive therapy including azathioprine and corticosteroids in order to limit the extent of the inflammatory process. Immunosuppressive therapy of multiple sclerosis, however, is only partially effective, and in most cases only offers a delay in disease progression despite antiinflammatory and immunosuppressive treatment.

Accordingly, there is a need for other therapeutics which are effective in the treatment of multiple sclerosis and other related diseases including those involving T-cell mediated damage to central or peripheral nerve tissue, such as peripheral neuritis, optical neuritis and amyotrophic lateral sclerosis.

It has now been found that an amino alcohol such as disclosed thereafter has a beneficial effect in the treatment of autoimmune diseases such as amyotrophic lateral sclerosis (Lou Gehrig's disease) or uveitis.

D5 (WO 2005/014525) describes S1P modulators with immunosuppressive efficacy which carry a rigid biphenyl- or, in some instances, a rigid biaryl-core structure. The three dimensional molecular motifs of the compounds of D5 are therefore quite remote to the compounds of the instant invention.

### Embodiments

1. The invention therefore provides a compound of formula Ia, 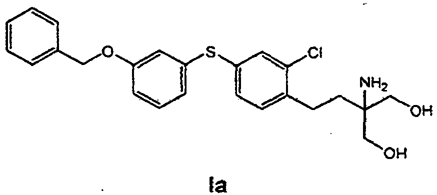 or a pharmaceutically acceptable salt thereof,
   or a phosphate derivative thereof of the formula: 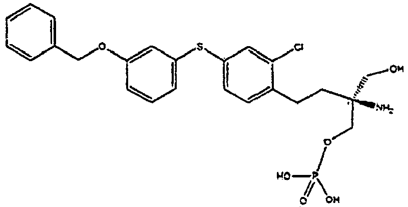 or 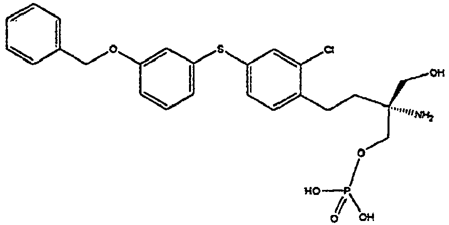 or a pharmaceutically acceptable salt thereof, as the sole active ingredient for use in the treatment of amyotrophic lateral sclerosis or uveitis.
2. The invention further provides a compound for use according to embodiment 1, which is a compound of formula Ia: 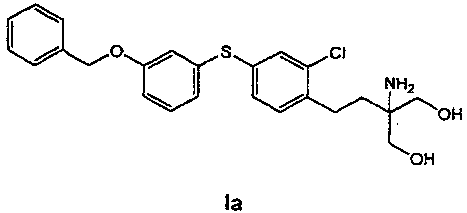 or a pharmaceutically acceptable salt thereof.
3. The invention further provides a compound for use in accordance to embodiment 1, which is a compound of the formula: 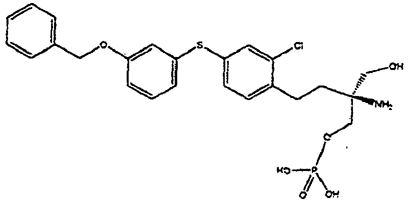 or 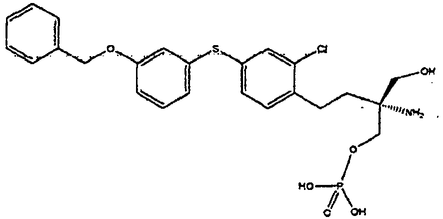 or a phartnaceutically acceptable salt thereof.
4. The invention also provides a compound for use in accordance to embodiment 2, wherein the treatment is treatment of uveitis.
5. The invention also provides a pharmaceutical composition comprising a compound of formula Ia or a phosphate derivative thereof or a pharmaceutically acceptable salt thereof as the sole active ingredient, as defined in embodiment 1, together with one or more pharmaceutically acceptable diluents or carriers therefore, for use in a treatment as defined in embodiment 1.

Phosphorylated derivatives of compounds of formula (Ia), e.g., phosphoric acid mono-2-amino-2-[4-(3-benzyloxyphenylthio)-2-chlorophenyl]ethyl-propyl] ester, can be prepared utilizing the procedures for synthesizing phosphorylated compounds described e.g., in WO 2005/021503 (see, e.g., pages 11 and 12). Optically active compounds of the phosphorylated derivatives of formula (Ia) can be prepared in high purity utilizing the procedure described, e.g., in Hinterding et al., Synthesis, Vol. 11, pp.1667-1670 (2003). As an example, an optically active compound of structural formula (Ia), phosphoric acid mono-2-amino-2-[4-(3-benzyloxyphenylthio)-2-chlorophenyl]ethyl-propyl] ester, can be prepared as described in the scheme below utilizing the procedures of Hinterding et al. (2003) *supra.* 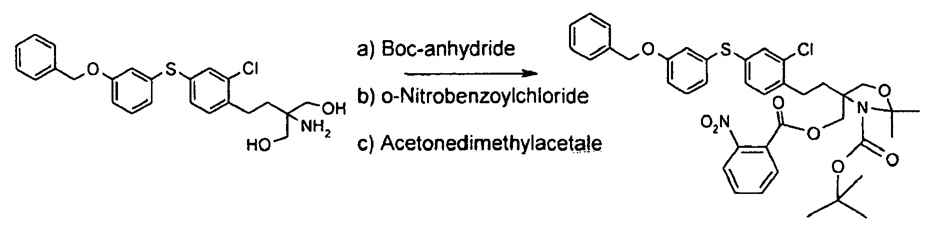 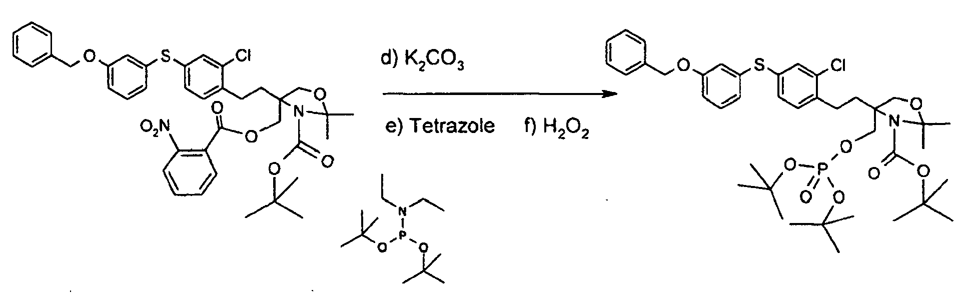
a) 1 equivalent of compound 1 and 1.2 equivalents Boc-anhydride in dioxane/acetonitrile or DMF/water (depends on solubility) + 1.2 equivalents NaOH 1 M in water (RT, overnight).
b) 1 equivalent of step a), 1.5 equivalents 2-nitrobenzoylchloride and 1.6 equivalents pyridine in CH₂Cl₂ (RT, overnight).
c) 1 equivalent of step b), 3 equivalents acetonedimethylacetale and 0.1 equivalents p-TsOH·H₂O in toluene (95°C, 3 hours).
d) 1 equivalent of step c) and 0.075 equivalents K₂CO₃ (powder) in MeOH/THF (1/1) (RT, 4 hours).
e) 1 equivalent of step a), 6 equivalents tetrazole (recrystallized from toluene or 0.45 M in CH₃CN) and 2 equivalents di-*t*-butyldiethylphosphoramidite in dry THF (RT, 3 hours).
f) 5 equivalents H₂O₂ (30%) directly into the reaction mixture of step e) (0°C, 1 hour).
Isolation: the reaction mixture is quenched with sodium thiosulfate (saturated in water) and extracted with ethyl acetate (3 x).

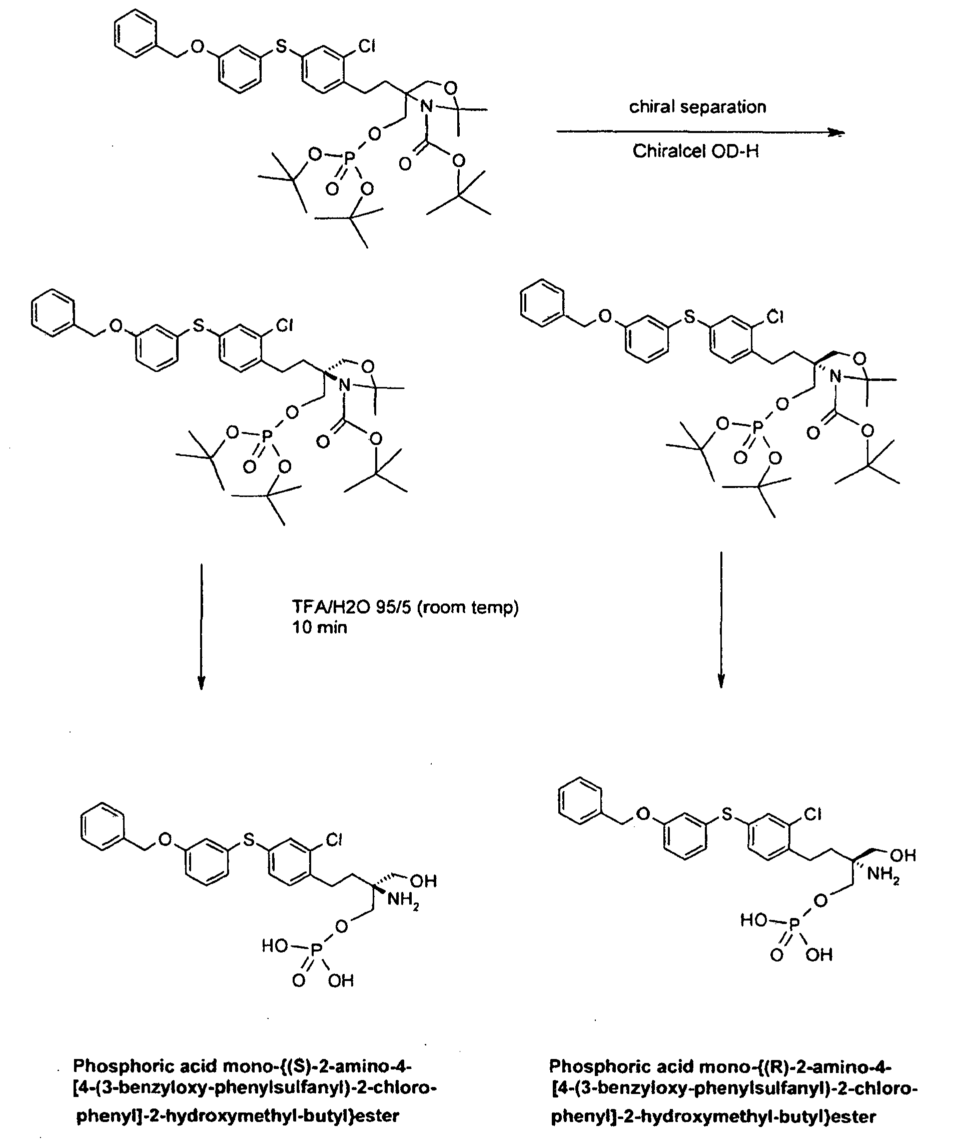

The compounds of the invention may exist in free or salt form, or as a hydrate. Examples of pharmaceutically acceptable salts of the compounds of the invention include salts with inorganic acids, such as hydrochloride and hydrobromide, salts with organic acids, such as acetate, trifluoroacetate, citrate, tartrate, methanesulfonate salts.

When the compounds of the invention have one or more asymmetric centers in the molecule, such compounds are to be understood as embracing the various optical isomers, as well as racemates, diastereoisomers and mixtures thereof.

The term "effective amount" refers to an amount of a compound of the invention which, when administered to the patient, is effective to treat an autoimmune disease, namely amyotrophic lateral sclerosis (Lou Gehrig's disease) and uveitis. With respect to treatment of an autoimmune disease this includes a reduction of symptoms of the disease, and any other indicators known in the art which show the treatment of the autoimmune disease.

Utility of the compounds of the invention in treating the diseases, disorders or conditions as hereinabove specified, may be demonstrated in animal test methods as well as in clinic, for example in accordance with the methods hereinafter described. The most widely used animal model for multiple sclerosis is experimental autoimmune encephalomyelitis (EAE), based on shared histopathological and clinical features with the human disease.

### Methods

**Animal models:** The monophasic model of acute experimental autoimmune encephalomyelitis (EAE) and the chronic relapsing form are considered to be instructive animal models for multiple sclerosis. EAE can be induced in susceptible animals by a single injection of CNS tissue or MBP emulsified in complete Freund's adjuvant into the base of the tail. A monophasic acute paralytic disease appears in susceptible rat strains, e.g., Lewis, Wistar rat, about 8-11 days post-sensitization. The symptomatic rats recover within the following 7 days, but in other species the attack is usually lethal. In the chronic relapsing disease models rats undergo one to three relapses following the acute disease bout. These relapses are usually from very mild to severe and are observed within 20-100 days after the acute bout.

### 1. Acute EAE model

Female Lewis rats are immunized by intracutaneous injection in the hind-paws with 0.1 mL of a mixture of guinea pig spinal cord and complete Freund's adjuvant [Difco H37 RA] (3.5 g guinea pig spinal cord + 3.5 mL 0.9% NaCl + 105 mg M. tuberculosis [Difco H37 RA] + 7 mL CFA (Difco H37 RA). Five-ten rats per group are used and somatic symptoms are judged daily on a scale of 0-3. The number of diseased animals as well as the time of onset of the disease is recorded. Test compounds, e.g. [Compound A: 2-amino-2-[4-(3-benzyloxyphenylthio)-2-chlorophenyl]ethyl-propane-1,3-diol; Compound B: (R)-2-amino-4-[4-(3-benzyloxyphenylthio)-2-chlorophenyl]-2-methylbutane-1-ol; and Compound C: (R)-2-amino-4-[4-(3-benzyloxyphenylthio)-2-chlorophenyl]-2-ethylbutane-1-ol) are administered daily from days 0-13 days by oral gavage. The statistical significance between treated and untreated groups is analyzed on each day using ANOVA analysis of variance followed by Dunn's multiple comparisons. In the absence of drug treatment symptoms of the disease (paralysis of the tail and both hind legs) usually develop within 8-11 days.

### Clinical grades:

1 = loss of tail tonicity
2 = weakness of one or both hind legs, or mild ataxia
3 = severe ataxia or paralysis accompanied by urinary incontinence

As shown in Table 1 below, Compounds A, B and C lead to prevention of disease symptoms when administered at doses between 0.1 and 10 mg/kg/day in this model.

**Table 1**

| **Compound** | **Dose mg/kg p.o.** | **Number of Animals with EAE/total** | | | **First symptoms on day** |
|---|---|---|---|---|---|
| | | Day 12 | Mean score in | Day 14 | |
| | | | control | | |
| Control | - | 5/5 | severity = 2.8 | | 9 |
| Compound A | 0.1 | 0/5 | | 3/4 | 9 |
| | 0.3 | 0/5 | | 1/5 | 14 |
| | 1 | 0/5 | | 0/5 | > 14 |
| Control | - | 5/5 | severity = 3 | | 10 |
| Compound B | 1 | 0/5 | | 0/5 | > 14 |
| Compound C | 1 | 0/5 | | 0/5 | > 14 |

| | | | | | |
|---|---|---|---|---|---|
| Severity = clinical grades 0-3 | | | | | |

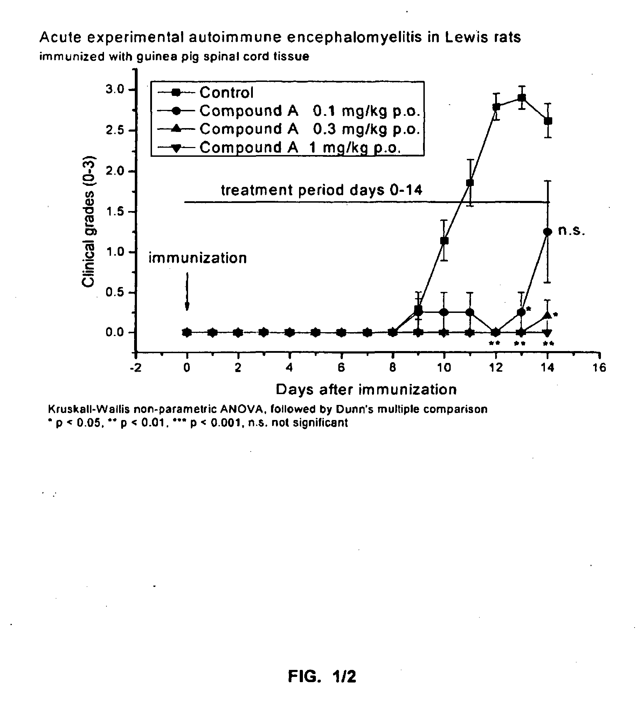

As shown in Figure 1, Compound A prevents disease symptoms when administered orally at doses between 0.1 and 10mg/kg/day in this model

Figure 1 shows the dose response effect of Compound A on prevention of disease symptoms in the acute EAE model.

### 2. Chronic-Relapsing EAE Model

Chronic-relapsing EAE is induced by injecting an emulsion of guinea pig spinal cord in complete Freund's adjuvant in the hind paws of Lewis rats. Six to ten rats per group are used and somatic symptoms are judged daily on a scale of 0-3. The number of diseased animals as well as the time of onset of the disease is recorded. Treatment with the test compound, e.g. a compound of the invention, e.g. Compound A as defined *supra,* is started on day 16 (after first disease bout) and continued until day 31. The statistical significance between treated and untreated groups is analyzed on each day using ANOVA analysis of variance followed by Dunn's multiple comparisons. In the absence of drug treatment 80-100% of the sensitized rats show clinical relapses during the first 40 days following immunization.

### Clinical grades:

1 = loss of tail tonicity
2 = weakness of one or both hind legs, or mild ataxia
3 = severe ataxia or paralysis accompanied by urinary incontinence

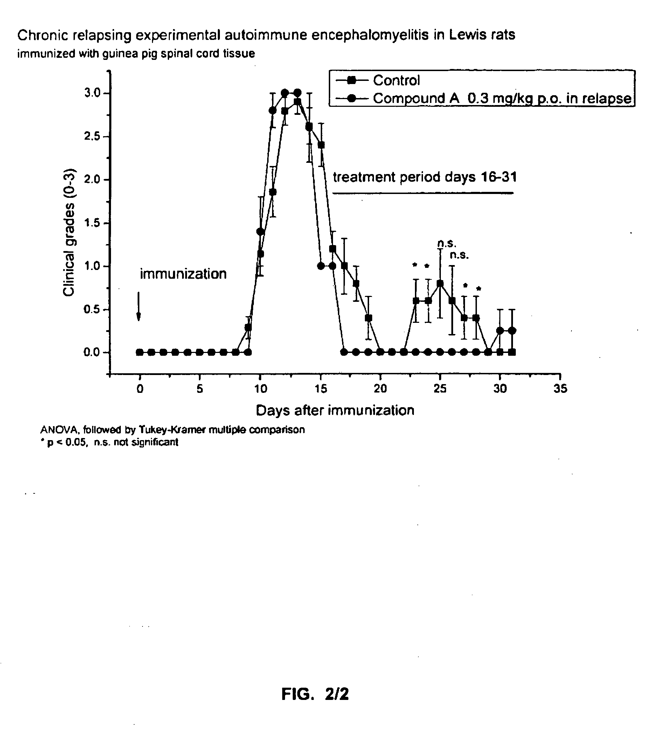

As shown in Figure 2, Compound A prevents clinical relapses when administered orally at a doses of 0.3mg/kg/day in the chronic relapsing EAE model. Figure 2 shows the effect of Compound A on prevention of disease symptoms in the chronic relapsing EAE model.

It is expected that similar results obtained with Compound A would be observed for Compounds B and C.

### 3. Chronic EAE Model

Induction of AEA in DA rat is induced as described by Lorentzen et al, 1995, J. Neuroimmunol.; 63(2):193-205 and Adelmann et al, 1995, J. Neuroimmunol.; 63(1):17-27. Briefly, rats are immunized with a mixture of DA rat brain and DA rat and bovine spinal cord homogenate supplemented with 0.02 µg/ml purified recombinant rat MOG protein. The mixture is homogenized and then mixed 1:1 with complete Freund's adjuvant containing 4 mg/ml M. *tuberculosis* H37RA(CFA). The resultant mixture is then homogenized using a Polytron PT3100 homogenizer (Kinematica, Lucerne, Switzerland). Rats are then injected subcutaneously at the dorsal root of tail with a single injection of 200 µl antigen/CFA. The resultant chronic disease is evaluated using numeric scale of progressive paralysis: 0, no paralysis; 1, loss of tail tonicity; 2, hindlimb weakening or ataxia; 3, hindlimb paralysis with or without urinary incontinence; 4, hindlimb and forelimb paralysis; 5, moribund or death. Clinical scores are evaluated on a daily basis, while body weight is determined every other day. At the peak of clinical disease, prior to treatment, animal groups are rearranged such that the clinical disease scores are comparable. Treatment of animals begins at the peak clinical disease on the 12^{th} day and continues daily the 33^{rd} day post-immunization (total 22 days). The test compound or vehicle (for the control groups) is administered orally daily.

In this assay, Compound A administered orally at a dose of 0.3, 0.1 or 0.03 mg/kg/d effectively inhibits chronic EAE. Statistically analysis demonstrates significant reduction in clinical disease at each dose of Compound A compared to that of the vehicle group.

### Clinical Trial

Suitable clinical studies are, e.g., open-label, dose-escalation or randomized, double-blind studies in patients with the aforementioned amyotrophic lateral sclerosis and uveitis. The beneficial effects on these autoimmune diseases, can be determined directly through the results of these studies which are known as such to a person skilled in the art. Such studies may also be suitable to compare the effects of a monotherapy using compounds of the invention as active ingredient and a combination of such compounds with a second drug substance.

The general clinical state of the patient is investigated weekly by physical and laboratory examination. Disease state and changes in disease progression are assessed every 2 months by radiological examination (MRI) and physical examination. Initially patients receive treatment for 2 to 6 months. Thereafter, they remain on treatment for as long as their disease does not progress and the drug is satisfactorily tolerated.

Main variables for evaluation: Safety (adverse events), standard serum biochemistry and hematology, magnetic resonance imaging (MRI).

Daily dosages required in practicing the present invention will vary depending upon, for example, the compound used, the host, the mode of administration and the severity of the condition to be treated. A preferred daily dosage range is about from 0.1 to 100 mg as a single dose or in divided doses. Suitable daily dosages for patients are on the order of from e.g. 0.1 to 50 mg p.o. The compound may be administered by any conventional route, in particular enterally, e.g. orally, e.g. in the form of tablets, capsules, drink solutions, nasally, pulmonary (by inhalation) or parenterally, e.g. in the form of injectable solutions or suspensions. Suitable unit dosage forms for oral administration comprise from ca. 0.1 to 30 mg, usually 0.25 to 30 mg active ingredient, e.g. from about 0.1 - 5 mg, together with one or more pharmaceutically acceptable diluents or carriers therefore.

Compounds of the invention may be administered by any conventional route, in particular, enterally, e.g., orally, e.g., in the form of tablets or capsules, or parenterally, e.g., in the form of injectable solutions or suspensions, topically, e.g., in the form of lotions, gels, ointments or creams, or in a nasal or a suppository form. Phosphate derivatives of the compounds of the invention are preferably administered parenterally. Pharmaceutical compositions comprising such compounds in free form or in pharmaceutically acceptable salt form in association with at least one pharmaceutical acceptable carrier or diluent may be manufactured in conventional manner by mixing with a pharmaceutically acceptable carrier or diluent.

The compounds of the invention may be administered in free form or in pharmaceutically acceptable salt form, e.g., as indicated above. Such salts may be prepared in conventional manner and exhibit the same order of activity as the free compounds.

Compounds of the invention are administered as the sole active ingredient for the treatment of the afore-mentioned autoimmune disorders.

Accordingly, in yet a further aspect, there is described :
5. A method comprising co-administration, e.g. concomitantly or in sequence, of a therapeutically effective non-toxic amount of a compound of the invention or a pharmaceutically acceptable salt thereof, and at least a second drug substance.
6. A pharmaceutical combination, e.g. a kit, comprising a) a first agent which is a compound of the invention or a pharmaceutically acceptable salt thereof, and b) at least a second drug substance, e.g. as indicated above. The kit may comprise instructions for its administration.

The terms "co-administration" or "combined administration" or the like as utilized herein are meant to encompass administration of the selected therapeutic agents to a single patient, and are intended to include treatment regimens in which the agents are not necessarily administered by the same route of administration or at the same time.

The term "pharmaceutical combination" as used herein means a product that results from the mixing or combining of more than one active ingredient and includes both fixed and non-fixed combinations of the active ingredients. The term "fixed combination" means that the active ingredients, e.g., a compound of the invention and a second drug substance, are both administered to a patient simultaneously in the form of a single entity or dosage. The term "non-fixed combination" means that the active ingredients, e.g., a compound of the invention and a second drug substance, are both administered to a patient as separate entities either simultaneously, concurrently or sequentially with no specific time limits, wherein such administration provides therapeutically effective levels of the 2 compounds in the body of the patient. The latter also applies to cocktail therapy, e.g., the administration of 3 or more active ingredients.

## Claims

1. A compound of formula Ia, 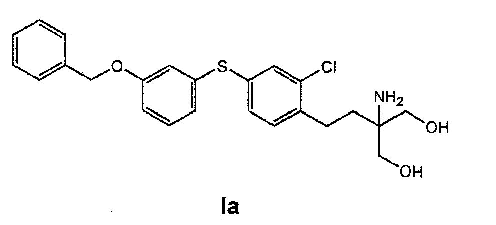 or a pharmaceutically acceptable salt thereof,
or a phosphate derivative thereof of the formula: 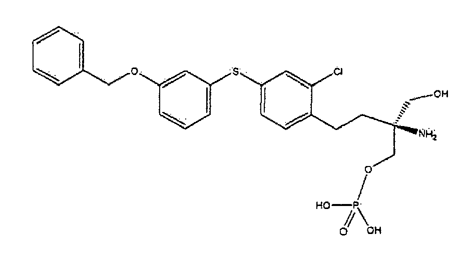 or 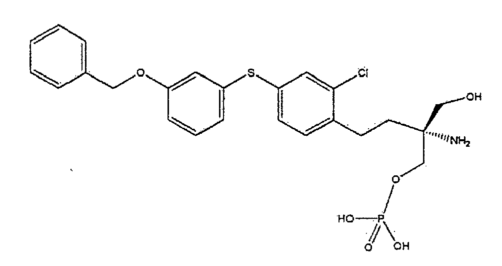 or a pharmaceutically acceptable salt thereof for use as the sole active ingredient in the treatment of amyotrophic lateral sclerosis or uveitis.

2. A compound for use according to claim 1, which is a compound of formula Ia: 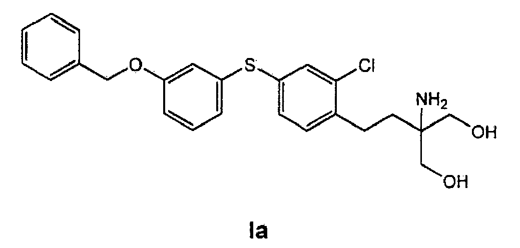 or a pharmaceutically acceptable salt thereof.

3. A compound for use in accordance to claim 1, which is a compound of the formula: 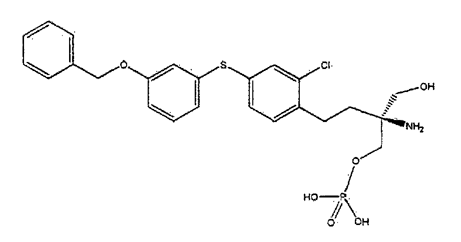 or 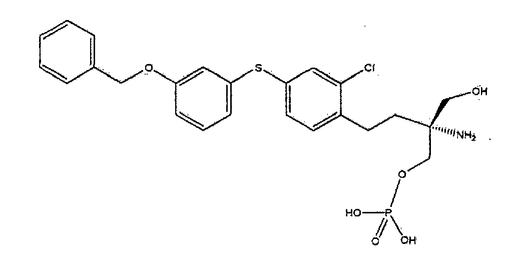 or a pharmaceutically acceptable salt thereof.

4. A compound for use in accordance to claim 2, wherein the treatment is treatment of uveitis.

5. A pharmaceutical composition comprising a compound of formula Ia or a phosphate derivative thereof or a pharmaceutically acceptable salt thereof as the sole active ingredient, as defined in claim 1, together with one or more pharmaceutically acceptable diluents or carriers therefore, for use in a treatment as defined in claim 1.

## Patentansprüche

1. Verbindung der Formel Ia 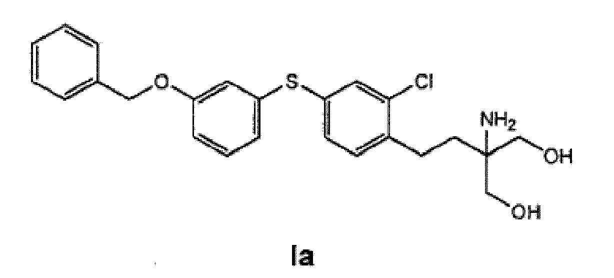 oder ein pharmazeutisch unbedenkliches Salz davon
oder ein Phosphatderivat davon der Formel: 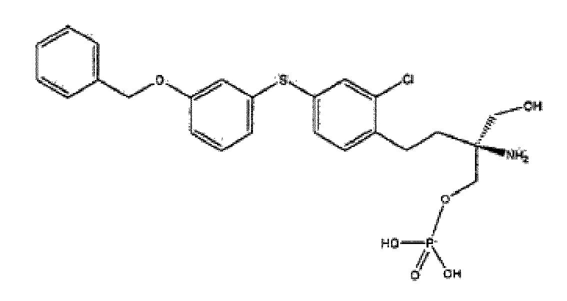 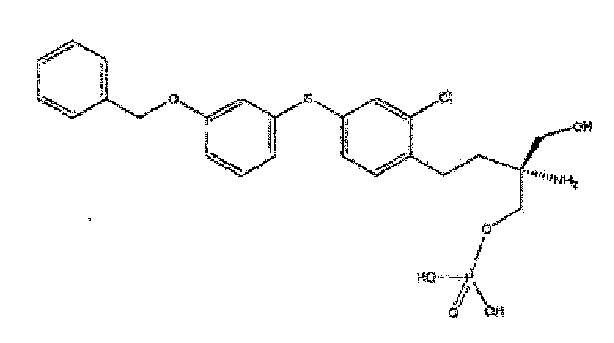 oder ein pharmazeutisch unbedenkliches Salz davon zur Verwendung als alleiniger Wirkstoff bei der Behandlung von amyotropher Lateralsklerose oder Uveitis.

2. Verbindung zur Verwendung nach Anspruch 1, die eine Verbindung der Formel Ia ist: 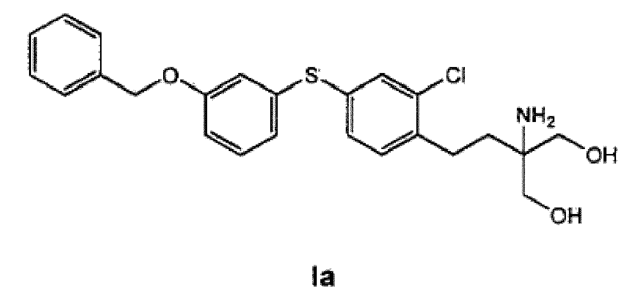 oder ein pharmazeutisch unbedenkliches Salz davon.

3. Verbindung zur Verwendung nach Anspruch 1, die eine Verbindung der folgenden Formel ist: 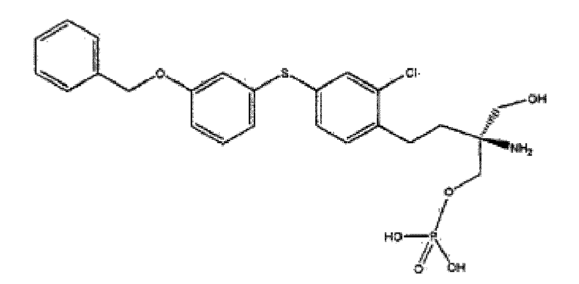 oder 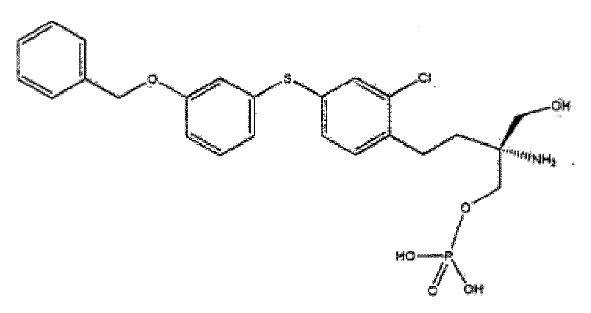 oder ein pharmazeutisch unbedenkliches Salz davon.

4. Verbindung zur Verwendung nach Anspruch 2, wobei die Behandlung die Behandlung von Uveitis ist.

5. Pharmazeutische Zusammensetzung, die eine Verbindung der Formel Ia oder ein Phosphatderivat davon oder ein pharmazeutisch unbedenkliches Salz davon als alleinigen Wirkstoff, wie in Anspruch 1 definiert, gemeinsam mit einem oder mehreren pharmazeutisch unbedenklichen Verdünnungsmittel(n) oder Trägem dafür umfasst, zur Verwendung bei einer Behandlung, wie sie in Anspruch 1 definiert ist.

## Revendications

1. Composé de formule Ia : 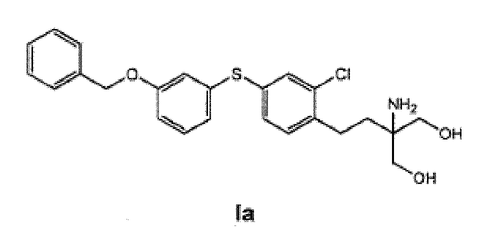 ou un sel pharmaceutiquement acceptable de celui-ci,
ou un dérivé phosphate de celui-ci de la formule : 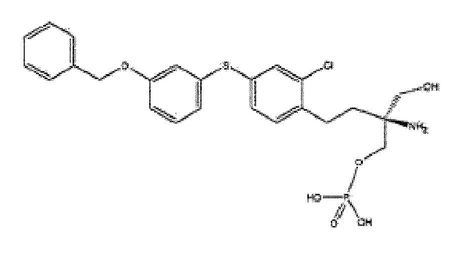 ou 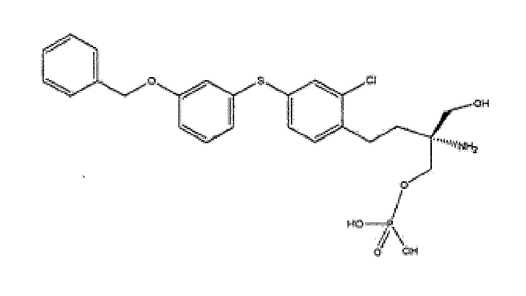 ou un sel pharmaceutiquement acceptable de celui-ci, pour une utilisation comme principe actif unique dans le traitement de la sclérose latérale amyotrophique ou de l'uvéite.

2. Composé pour une utilisation selon la revendication 1, qui est un composé de formule Ia : 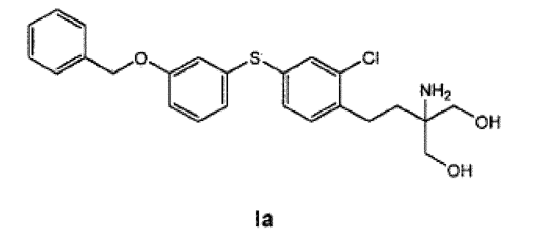 ou un sel pharmaceutiquement acceptable de celui-ci.

3. Composé pour une utilisation selon la revendication 1, qui est un composé de la formule : 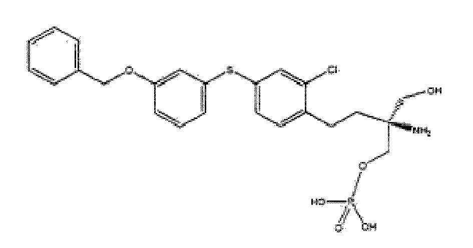 ou 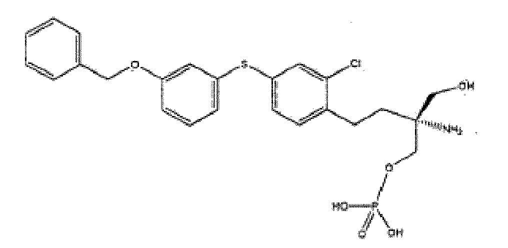 ou un sel pharmaceutiquement acceptable de celui-ci.

4. Composé pour une utilisation selon la revendication 2, dans lequel le traitement est un traitement de l'uvéite.

5. Composition pharmaceutique comprenant un composé de formule Ia ou un dérivé phosphate de celui-ci ou un sel pharmaceutiquement acceptable de ceux-ci comme principe actif unique, tel que défini dans la revendication 1, conjointement avec un ou plusieurs diluants ou supports pharmaceutiquement acceptables à cet effet, pour une utilisation dans un traitement tel que défini dans la revendication 1.
